# EUROPEAN PATENT APPLICATION

(11) **EP 4 282 389 A1**
(43) Date of publication of application: **29.11.2023**
(21) Application number: 22175140.7
(22) Date of filing: 24.05.2022
(51) Int. Cl.: A61F 7/02, A61F 7/00, B33Y 80/00

(54) **SYSTEM ADAPTED FOR PROVIDING A THERMAL AND THERAPEUTICAL EFFECT AT A SPECIFIC POINT OF BODY OF A PATIENT**

(71) Applicant: CryoThermo 4.0 Sàrl, 2822 Courroux (CH)
(72) Inventor: von Gunten, Marco, 2905 Courtedoux (CH)
(74) Representative: P&TS SA (AG, Ltd.)

(57) **Abstract**

The present invention relates to a system (1) adapted for providing a thermal and therapeutical effect to a patient (10) at a specific point of his body. The system comprises an element of contact (20a, 20b), adapted to be placed in contact to the point of body. The element of contact comprises a structural element (21) defining a tridimensionality shape. The system also comprises a thermal regulation device (50) and a regulation fluid circulating through the thermal regulation device and the element of contact. The present invention also relates to a process adapted to manufacture such a casing.

## Description

### Technical domain

The present invention concerns a device such as a thermal device being used or usable as a compress, in particular a thermal compress. The present device is adapted to be placed on the body of a user or a patient, preferably at a specific location on the body such as a muscle, ligament, tendon or an articulation like a knee, a shoulder or a elbow. The present device is made of, or comprises, a material adapted to deliver a predetermined temperature toward such a specific point of the body. Such predetermined temperature refers to a hot temperature or a cold temperature or a cyclic temperature. The device can comprise additional functions such as electrostimulation and/or infrared irradiations as well medicine substances under form cream, liquid or gel.

### Related art

Thermal compresses are already known. They are usually flexible pieces comprising a thermal gel, which are put on a part of the body where a muscular or articulation pain is sensed, or to improve physical recovery after an effort. They are often used by sportive people, or people having back pain or articular pain. Such thermal compresses need to be placed in an oven/micorowaves or in a cold storage, such as a fridge or a freezer, prior to be used on the body. This requires some time before the patient can benefit from its warm or cold effect. In addition, the effect of the known thermal compresses, once placed on the body, is limited over time, since its temperature equilibrates with the environmental temperature. Thus, the thermal benefit is unregular and may be disappointing. In addition, the compress can be too hot or too cold when placed on the body, resulting in a discomfort.

Another drawback of the known thermal compresses is that their shape is often not adapted to the portion of the body on which they are placed. The patient often needs to maintain it with his own hand, or to remain at rest, which limits his movements and activities.

There is thus room to improve the known thermal compresses from a thermal, therapeutic, medical and morphological point of view.

### Short disclosure of the invention

An aim of the present invention is the provision of a system or arrangement that overcomes the shortcomings and limitations of the state of the art. It is in particular an aim to provide a thermal arrangement with a longer lasting effect than a traditional compress, a more regular and constant temperature, or more adapted temperature, or a variable temperature or a combination thereof.

Another aim of the invention is to provide a system or an arrangement, in particular based on thermal exchanges, adapted to the morphology of the user or the patient at a specific point of body. This provides the advantage that the patient remains free of his movements, or is at least not forced to inactivity.

Another aim is to provide a method to produce a system or an arrangement, in particular based on thermal exchanges. It is in particular an aim to provide a fast and convenient process allowing to produce such a system or part of it on demand.

Additional advantages will become apparent through the detailed description below.

According to the invention, these aims are attained by subject-matter of the independent claims, object of the present invention. The details are object of the dependent claims.

The system or the arrangement according to the present description comprises an element of contact which can be used as a compress, and which allows thermal exchanges, in particular controlled thermal exchanges with the body at a specific point of the body. The element of contact according to the present invention may have a three-dimensional shape complementary to the specific point of body of the patient, so that it can easily fit with the morphology of the patient.

It can for example comprise a rigid or semi-rigid structural element such as a casing, a beam assembly, a grid, or any convenient structural element, adapted to provide and maintain a suitable tridimensionality shape, so that comfort is increased. The structural element defines a hollow space or a skeleton or an armature which is also adapted to support additional elements such as internal pipes, electrical wires, reservoirs of substances having health benefits such as a cream, a gel or a liquid. The structural element has a surface of contact (21a) adapted to be in contact with the skin of a patient. The surface of contact can be provided, at least partially, with a soft material such as a fabric, soft polymer or any material allowing to improve the comfort of the patient and the thermal transfer at the specific point of the body. Alternatively, the structural element can be inserted in a pouch or a bag for a more comfortable application on the body. Such a pouch or bag may comprise a gel or be filled with bids of material adapted to transfer thermal energy. The structural element can be made of a rigid or semi-rigid material so as to maintain its tridimensionality shape.

The system or arrangement according to the present description can also comprises an external (dissociated) thermal regulation device, adapted to produce and maintain a predetermined constant temperature at the element of contact. The thermal regulation device can be connected to the element of contact by means of pipes so as to allow a fluid circulating within or along the structural element of the element of contact, while being regulated by the thermal regulation device.

The system or arrangement can be provided with one or more sensors, such as thermo-sensors, heart rate sensor, blood pressure sensor, skin temperature sensor, or other bio-sensors. In particular, the element of contact, more particularly, the surface of contact of the element of contact is preferably provided with at least one thermo-sensor.

The system or arrangement according to the present disclosure preferably comprises a control unit, adapted to activate or deactivate the circulation of one fluid or more than one fluid. The control unit can in addition control the temperature of such circulating fluids, at a predetermined value, or between several predetermined values, as well as the variation of such temperatures such as increase or decrease rate of temperature or cyclic variation of temperatures. In case several fluids are circulating, the temperature and temperature variation of each of these fluids can be independently controlled.

The system or arrangement according to the present description may be provided with one or a combination of several different additional features depending on the needs. For example, it can be provided with communication means so as to be connected to a remote electronic device and communicate data therewith, including control data to pilot the thermal regulation device and/or data sensed by one or more sensors.

The system or arrangement of the present description may in addition comprise one or several storage compartment or reservoirs, combined with one or several porous areas adapted to deliver some quantities of biologically active compounds or mixtures to a specific point of the body. By this way, a substance can be delivered to the skin in contact with the element of contact.

The thermal regulation device comprises one or both heating device and/or cooling device so that the requested temperature can be produced. It further comprises the necessary elements such as pump and compressor, as well as all necessary features adapted to provide heat or cold. It can comprise at least one cold generation device such as Peltier device or chemical Peltier effect for cooling a circulating fluid. In addition, or alternatively, it can comprise at least one warm generation device such as Peltier devices, resistors, etc for warming a circulating fluid. A control unit is adapted to maintain the temperature at a predetermined constant value, to increase or decrease the temperature according to a predetermined rate, or to vary the temperature according to predetermined cycles.

The system or arrangement according to the present description may in addition comprise one or several tracking means adapted to determine and follow the status of the product (compress) or of the application (usage). Tracking means can take the form of a RFID or any other suitable device.

The structural element of the element of contact may be sufficiently rigid so as to be used as a fixing element, for example in case of broken bones. The present system or arrangement can thus be used as a splint, a plaster cast, or in combination with such a splint or plater cast. The present system or arrangement can also be used for examples as a knee pad, or an elbow pad, or in combination with such a knee pad or elbow pad.

The present system or arrangement may be used by any individual either at home or in the framework of medical environment such as an hospital, a physiotherapeutic office. It can be used either alone or in combination with other treatment or medical devices. As an example, the present system or arrangement can be used by a sport practitioner or professional workers after an intense physical activity so as to improve physical recovery or to relieve potential muscular, ligament, tendon pain or articular damage. It can be used alternatively before an intense physical activity, so as to prepare the body or part of the body to such an intense effort.

In the present disclosure, the term "specific point of the body" denotes any part of the body in necessity of attention or treatment. It can be a point of injury such as a body pain as luxation, a local inflammation, rheumatism, osteoarthritis, a sprain, a hernia or a bone fracture. It can alternatively be a point without declared injury or pain, but in need of preparation before an effort or in need of protection during an effort. As such, a specific point of body can denote an articulation such as a knee, an elbow, a wrist or a shoulder. It can also denote a muscle such as the knurl, a thigh or a bicep. The specific point of the body can in addition denote a region of the body, such as the back, the lumbar region or the cervical area.

In the present disclosure a "patient" denotes any person using the present system or arrangement to benefit from thermal and/or therapeutical effects at a specific point of his body. A patient may denote a person having an injury, a local inflammation or any pain necessitating a thermal treatment. A patient also denote any person having no specific pain but willing to prepare for a physical activity such as a sportive competition or a hard physical work. A patient can also denote a user aiming at a relaxing time with a thermal effect of the present system or arrangement.

With respect to what is known in the art, the invention provides the advantage of a more convenient and a better controlled device based on thermal exchanges.

### Short description of the drawings

Exemplar embodiments of the invention are disclosed in the description and illustrated by the following figures :
- Figure 1 : overview of the arrangement according to one example of the present disclosure
- Figure 2A : details of a compress according to an embodiment of the present disclosure
- Figure 2B : details of a compress according to another embodiment of the present disclosure
- Figure 3: transversal view of a compress according to an embodiment of the present disclosure

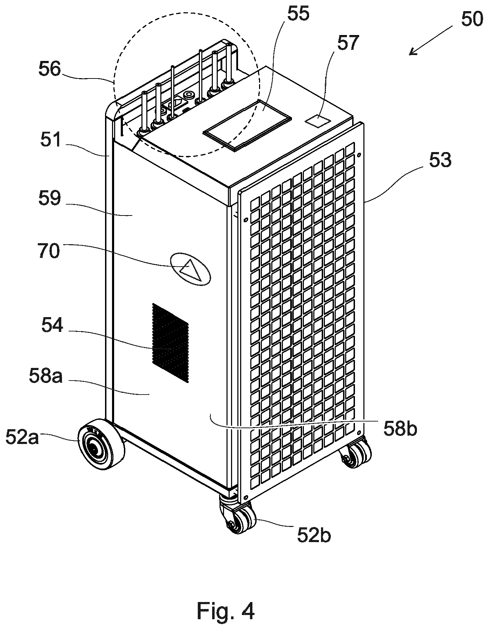

- Figure 4: thermal regulation device view according to an embodiment of the present invention
- Figure 5: details thermal regulation device according to an embodiment of the present disclosure.
- Figures 6A, 6B, 6C: details of the elements of contact according to embodiments of the present disclosure.

### Examples of embodiments of the present invention

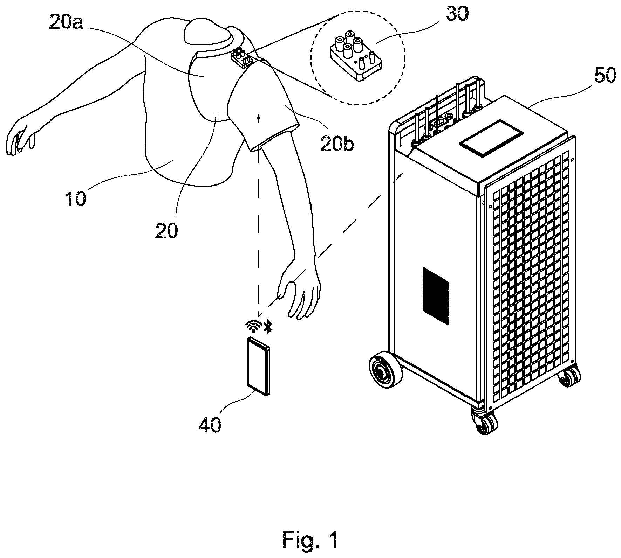

With reference to figure 1, the system **1** according to the present disclosure comprises one or more element of contact **20.** Such an element of contact **20** is adapted to be placed in contact or close to a specific point of the body of a patient **10,** preferably in direct contact with the skin. The element of contact **20** of the present system **1** is adapted to fit with the body of the patient, at a specific point of its body. This means that the surface of contact adopts or reflects the tridimensionality shape of the body at a specific point or area.

Fixation points and/or additional fixation element such as one or more belt, strap, Velcro or clasp, buckle (not represented) can be arranged on the element of contact **20** to maintain it in close contact with the body. For example, a belt can be attached to opposite ends of the element of contact so as to be arranged around the abdomen of the patient **10.** Depending on the specific point of body, such a belt can be used around a leg or an arm. Alternatively, or in addition, the shape of the element of contact is adapted so that the element of contact can remain on the body by itself. For example, the element of contact **20** can have a concave shape allowing to surround a member such as a leg or an arm in a way that it remains in contact with the body by itself. The element of contact **20** can alternatively or in addition comprise several subparts **20a, 20b.** These subparts can be adapted to cooperate with each other. To this end, the subparts can exhibit complementary shapes adapted to combine them. Alternatively or in addition, arrangements such as snaps, belt, strap, Velcro, clasp, buckle (not represented) can be envisaged. Alternatively, the subparts can be integral elements which are articulated so as to be adapted to the morphology of the patient. To this end they can be linked by means of a hinge, such as a flexible polymer ore any other equivalent means. For example, it can comprise two half subparts each having a concave shape so that they can be arranged together around a member such as a leg or an arm, in a way to surround the corresponding member. The two half subparts **20a, 20b** can be joined together by any suitable means or combination of means such as a snap, a belt, a strip, a clip and related means. Such an arrangement may also be used in case of a fracture of a bone, in particular of a long bone such as a femur, an ulna or any other long bone. The present element of contact may be used in replacement of the traditional plaster or resins used to maintain the bone fragments.

Figure 1 shows an example of such an element of contact **20** having two subparts **20a** and **20b,** each one being adapted to the local shape of the body. For example, a first subpart **20a** is adapted to fit with the top of the shoulder and a second subpart **20b** is adapted to fit with top of the corresponding arm. The two subparts **20a, 20b** are designed so as to fit one with the other so as to provide an element of contact covering all, or substantially all, the region of the body in need of attention. Although the two subparts **20a, 20b** can be fixed together so as to provide globally one single rigid element of contact, they can also be arranged in a way to remain at least partly mobile with respect to one another. In the case of figure 1, the first subpart **20a** covering the top of the shoulder and the second subpart **20b** placed on the top of the corresponding arm may be joined by means of a flexible link such as an elastic so as to allow some moves of the arm independently from its shoulder. The connection may be rigid if necessary.

It is here understood that several subparts such as two, three, four or more can be used and assembled to cover the specific point of body in a more complete way. According to an embodiment, the present element of contact **20** can be designed as a modular assembly wherein each subpart has a predetermined shape adapted to cover a predetermined point of the body. The element of contact **20** is of course not necessary constituted by several subparts, as described here, but can consist on only one element.

An element of contact according to the present disclosure comprises at least one structural element **21** adapted to provide and maintain a predetermined tridimensionality shape.

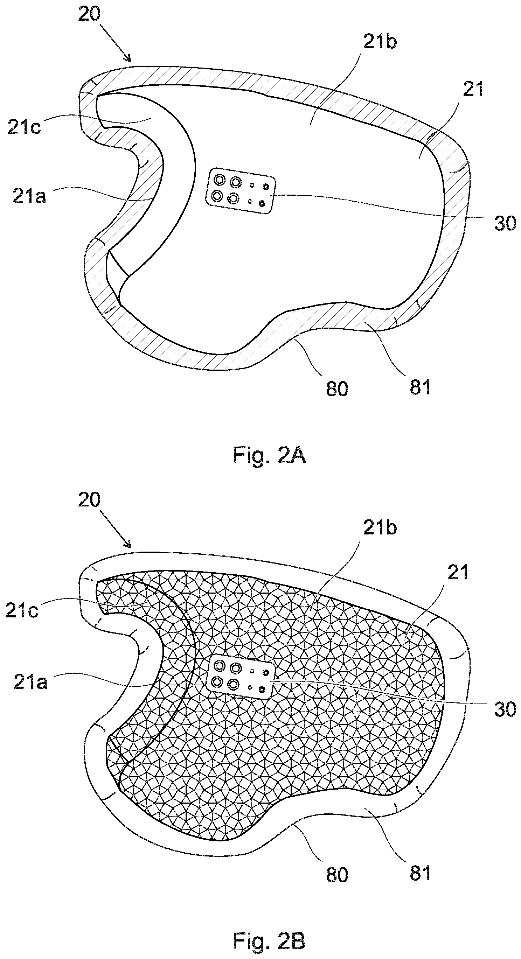

Figures 2A and 2B show an example of a structural element **21** according to an embodiment of the present disclosure. The structural element takes here the form of a casing **21C.** It comprises a surface of contact **21a** adapted to be oriented toward the body of the patient. The surface of contact **21a** may be concave so as to surround a body part having a convex shape, such as a leg, an arm or any other convex part of the body. However, depending on the body part to be considered, the surface of contact **21a** can have any adapted tridimensionality shape. For example, it can be flat or substantially flat, or even convex, to be applied for example on the back or behind the knee.

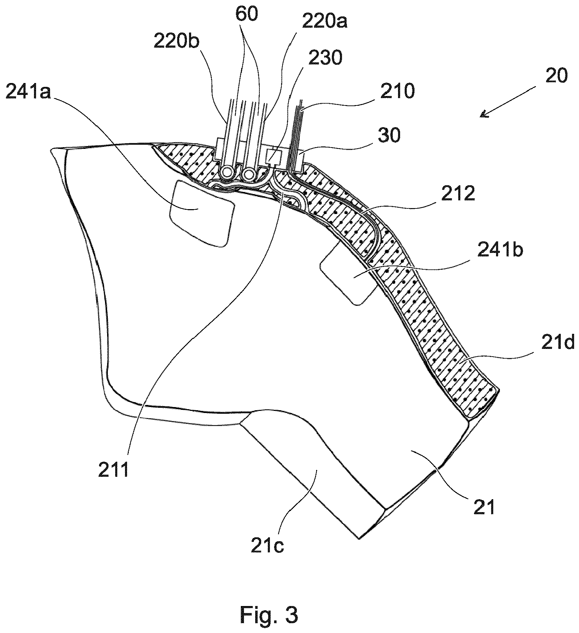

The structural element comprises a top surface **21b,** opposite the surface of contact **21a.** The top surface **21b** is thus not assumed to be in contact with the patient, but to remain oriented outwardly when the element of contact **20** is placed on the body. The top surface **21b** and the surface of contact **21a** can be combined through an edge 21c so as to define an internal hollow space **21d** (figure 3). According to an embodiment, the structural element **21** formed by the surface of contact **21a,** the top surface **21b** and the edge **21c** is completely hermetic so that the internal hollow space **21d** is waterproof. As such a liquid or a gel can be placed inside the structural element **21** of the element of contact **20** or can be circulated through dedicated connection points. Alternatively, the structural element **21** of the element of contact **20** is not waterproof as such but can comprise one or several waterproof internal pouch (not represented) placed inside the casing. Such an internal pouch may be hermetic and filled with a liquid or a gel or any suitable material which can be used as thermal storage or thermal transfer. Alternatively, the internal hollow space **21d** can comprise one or mor pipes wherein one or more fluid circulates. Beside the internal pouch or the pipes placed inside the structural element **21,** the internal hollow space **21d** may remain empty, meaning that the internal pouch, if any, is in direct contact with the internal surface of the structural element **21.** Alternatively, the internal space **21d** of the structural element **21** may be filled with a material adapted to conduct thermal energy from the internal pouch, if any, or from the internal pipes, if any. Thus, a thermal inertia can be obtained for a better and/or longer thermal effect.

According to another embodiment, one or more of the solid surfaces of the structural element, such as the surface of contact **21a,** the top surface **21b** or the edges **21c,** is replaced by beams or ribs or grids, so as to lighten the structural element without prejudice to its tridimensionality maintenance. As such, the structural element **21** can take the form of a skeleton **21S.**

The element of contact **20** further comprises at least one first fluid connection point **220a, 220b** adapted to receive a fluid or several distinct fluids inside the structural element **21.** According to an embodiment, a fluid can be input in the internal hollow space **21d** of the structural element **21,** either directly if the structural element is waterproof or through some internal pipes and/or internal pouch, so as to be stored inside the element of contact. If the structural element **21** denotes a waterproof solid surface, so as to provide a casing, it can comprise some tubular recesses within the material of the envelop, allowing the circulation of the fluid. Alternatively, when the structural element is a skeleton **21S,** the fluid can be circulated through internal pipes.

According to an embodiment, the fluid can be circulated through the structural element **21** being a casing **21C** or a skeleton **21S** in a continuous way or in a semi continuous way. To this end, at least two first fluid connection points **220a, 220b** are necessary. These first fluid connection points are each connected to a pipe, one being used as an input pipe and the other one being used as an output pipe. These pipes are connected to a regulation device **50** adapted to circulate the fluid through the pipes. The regulation device **50** comprises at least one pump **70,** such as a peristaltic pump, or equivalent circulating means adapted to circulate the fluid. Although the flow rate can be fixed and determined by construction, the thermal regulation device 50 can comprise a means to vary, adjust or adapt the flow rate according to the needs. For example, the speed of the pump **70** can be controlled to provide a regulated flow rate of the fluid, either constant, or varying according to predetermined parameters. In a semi-continuous way, the liquid can be circulated through the structural element **21** being a casing **21C** or a skeleton **21S** for a given time and stopped for a given time. It can then circulate again without removing or connecting pipes, when the temperature reaches a predetermined threshold for example.

According to a specific embodiment, the first fluid connection point 220a, 220b can be disconnected from the input and output pipes once a fluid is placed inside the element of contact. In this case, the patient can benefit from the thermal effect while being free of any fluid connection with the external thermal regulation device **50** at least for a certain time.

The thermal regulation device **50** is further provided with at least one thermal regulation element (58a, 58b) adapted to regulate the temperature of one or several circulating fluids **60.** Such a thermal regulation element preferably actively regulates the temperature of the fluid according to a predetermined program. It allows for example to maintain the temperature of the fluid at a predetermined value along a great time such as more than half an hour, more than an hour or even an indefinite time, until the thermal regulation device **50** or the corresponding thermal regulation element is stopped. The thermal regulation element can also be adapted to vary the temperature of one or several fluids according to a predetermined program. It can for example allow a continuous increase or a continuous decrease of the temperature between predetermine threshold values, with a predetermine temperature ramp. Alternatively, or in addition, the thermal regulation element can automatically adapt the temperature of the fluid according to one or more parameters values received from one or more sensors.

The at least one thermal regulation element can be adapted to produce heat so as to warm a fluid above the body temperature or above the environment temperature. The temperature of the fluid can thus be maintained at a value comprised between 30° and 50°, preferably between 40°C and 45°C. Alternatively, the at least one thermal regulation element can be adapted to produce cold so as to cold the fluid at a temperature lower than the body temperature or the environmental temperature. The temperature of the fluid can thus be decreased and maintained at a value comprised between -5°C and 10° or between 0°C and 5°C or between 2°and 3°C. It is understood that alternative range of temperatures can also be selected according to the needs.

The thermal regulation device **50** may comprise one of such thermal regulation elements, either for the cold or for the hot production. Preferably, the thermal regulation device **50** comprises two thermal regulation elements, one being a heat production element **58a,** dedicated to warm the corresponding regulating fluid and the other one being a cooling element **58b** dedicated to cool the corresponding regulating fluid. In that specific case, two distinct fluid circuits may be provided each one having its own circulating fluid and its own second fluid connection points **564a, 564b, 565a, 565b** on the thermal regulation device **50.** Alternatively, the regulation device **50** comprises only one thermal regulation element adapted to produce either cold or hot temperatures, so that only one circulating fluid circuit is necessary.

It is understood that the number of first fluid connection points **220a, 220b** can be adapted accordingly so that a warm fluid or a cold fluid or a combination of several fluids can be circulated through the element of contact **20.** It is also understood that in case of several subparts **20a, 20b,** each one of the several subparts is preferably provided with at least one first fluid connection point.

The circulating fluid **60** denotes here any thermal regulating fluid such as a heat-transfer fluid or a coolant, either liquid or gaseous. It can be based on liquid comprising glycols or any other equivalent heat transfer liquid.

The thermal regulation device **50** can be provided with a battery or any suitable rechargeable power storage. It can also be used directly through an electrical plug **562.** An additional power source can be optionally provided such as a solar panel **53.** The regulation device **50** may be provided with an air-exchange system **54** adapted to circulate air through the thermal regulation device **50.** Such an air-exchange system may comprise one or more fans, one or more grids and any suitable means to make air circulated.

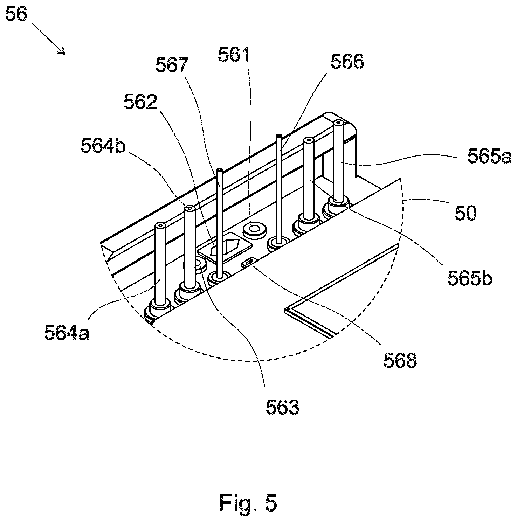

The thermal regulation device **50** can in addition be provided with an electrical stimulation system **59** adapted to deliver electrical stimuli to the patient through the element of contact **20.** To this end, the regulation device 50 is provided with one or several electrical connection means **566, 567** (Figure 5). The element of contact **20** is also provided with one or more suitable electrical connection means **210c, 210d** (figure 3), and corresponding stimulation plates **241a, 241b, 241c, 241d** or electrodes adapted to provide the electrical stimuli to the patient **10.** The necessary electrical cables **212** can be lodged in the internal hollow space of the element of contact **20.** The stimulation plates **241a, 241b, 241c, 241d** are located on the surface of contact **21a** so as to deliver the suitable stimuli to the body through the skin of the patient **10.** Although electrical stimulation is mentioned here, it can be completed or replaced by any other necessary stimulations such as ultrasounds stimulation, magnetic stimulation, light irradiation such as infrared light irradiation. For example, the local infrared irradiation may be used in addition to a warm circulating fluid to irradiate some very specific points of the body in contact with the surface of contact **21a** of the element of contact **20.** A temperature different than the temperature of the circulating fluid can thus be obtained at precise dedicated points.

The thermal regulation device **50** can be provided with a connection panel **56,** wherein all the fluid connection points and the electrical connection means are localized. The connection panel **56** can comprise any additional connection means that can be necessary. For example, it can comprise one or more 12V connection plug **561,** one or more 24V connection plug **563.** It can comprise one or several USB connection plug **568** or other type of data connection plugs such as an Ethernet connection plug. It can comprise a communication unit adapted to receive and/or send data through wi-fi or Bluetooth or any suitable communication protocol toward a remote device such as a smartphone or a computer. Furthermore, the regulation device **50** may be connected or connectable to a communication infrastructure such as 4G or 5G infrastructure.

The thermal regulation device **50** can in addition be provided with a command panel **57** allowing the user imputing data such as control data. For example, the user can select a program directly from its smartphone apps or from the command panel in a menu, or manually define a temperature, or a temperature variation or any other parameter of use of the regulation device **50.** The regulation device **50** may also be provided with a display **55** or any equivalent visualisation means. Alternatively, or in addition, the regulation device **50** is connected or connectable to an external command panel and/or an external display. Such an external command panel and/or display can take the form of any suitable computing device such as a personal computer, a tablet, a phone terminal. The command panel and the display cooperate with the dedicated command program, which may take the form of an application, either localized on the command panel or remotely localized on a server.

The thermal regulation device **50** may be mobile or portative so that the patient **10** can bring it with him when it moves. As such it can be brought at belt or via a shoulder strap. Depending on the equipment provided on the regulation device **50,** its weight can be more or less important. For better comfort, the regulation device **50** can be arranged on a mobile support **51** such as a frame provided with a handle. The mobile support **51** may be provided with some wheels, either one pair or two pairs of wheels **52a, 52b.** Alternatively, the thermal regulation device **50** can be fix and dedicated to an internal use, such as an hospital.

The thermal regulation device **50** is connected or connectable to a data computing unit **40,** adapted to receive some data from the regulation device **50** or from the element of contact **21a,** for example via the casing connection panel **30.** To this end, the element of contact **20** can be provided with one or several sensors such as thermal sensor, adapted to sense the temperature at the skin of the patient **10** or elsewhere it may be necessary. Additional sensors may be arranged on the patient or adapted to sense some physiological parameters of the patient **10,** such as heart frequency, blood pressure, body temperature. The computing unit **40** may be used as a control unit allowing to pilot the regulation device **50** in addition to, or instead of the panel control **57.**

The thermal regulation device **50** and/or the element of contact **20** can be provided with a tracking means such as an RFID label. Data related to the status of the regulation device **50** and/or the element of contact **20** can thus be retrieved and monitored.

According to an embodiment, the element of contact **20** is provided with one or several reservoirs **230** adapted to receive some substances such as cream, oil, liquid, gel. A reservoir **230** is for example lodged in the casing connection panel or in the internal hollow space **21d** of the structural element **21,** being a casing **21c** or a skeleton **21S,** or incorporated or supported by the structural element. The reservoir comprises a surface of contact with the skin of the patient **10** so that the substance it contains can be delivered to the skin of the patient **10.** Such a surface of contact can take the form of a porous surface or permeable surface allowing the substance to diffuse outside the reservoir **230.** The reservoir may be filled with such a substance from a dedicated closable aperture placed for example on top surface **21b** of the element of contact.

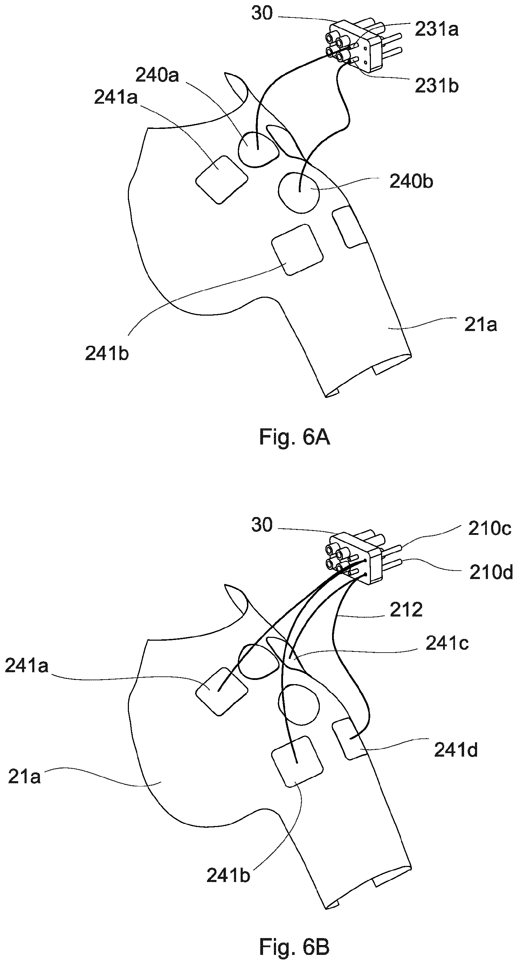

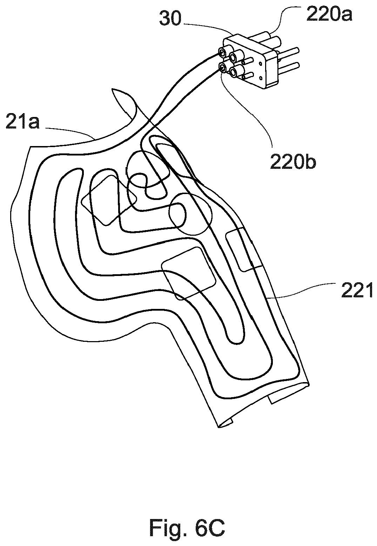

Referring back to figures 1 to 3, the element of contact **20** can be provided with a casing connection panel **30,** wherein all or most of the connection means are regrouped. The casing connection panel **30** can be included or embedded in the structural element of the element of contact **20.** It is preferably arranged at a position easily accessible when the element of contact is placed on the body of the patient **10.** It can be for example placed on the top surface **21b** or on the edge **21c** of the structural element. In case waterproofness is required, the necessary sealing is provided to join the casing connection panel **30** and the casing **21** in a hermetic way. The casing connection panel **30** can thus comprises at least one first fluid connection point **220a, 220b.** Figures 6A to 6C show examples of different connection modes related to the use of the element of contact **20,** wherein only the surface of contact **21a** is shown. The casing connection panel **30** comprises a first electrical connection means having two pins **210c, 210d.** The corresponding electrical cables **211** join these pins to some stimulation plate or electrodes **241a, 241b** positioned on the surface of contact **21a.** Figure 6B shows an example wherein the casing connection panel **30** comprises a second electrical connection means comprising two pins **210c, 210d** to connect stimulation plate or electrodes **214c, 241d.** An ensemble of several electrical cables **211** joins the two pins **210c, 210d** to a set of stimulating plates or electrodes **241a, 241b, 241c, 241d.** A different stimulation than the stimulation provided through the first connection means can thus be operated. Alternatively, one of the first and second electrical connection means **566, 567** can be used to transmit some parameters sensed by sensors placed on the surface of contact **21a** of the structural element **21,** being a casing **21C** or a skeleton **21S.** Figure 6C shows an example wherein the casing connection panel **30** comprises a fluid connection point comprising two first fluid connection means **220a, 220b** each adapted to receive a pipe, and an internal pipe **221** in contact or close to the surface of contact **21a** of the structural element **21.** The fluid delivered by the regulation device **50** can thus circulate through the element of contact and provide the requested thermal effect to the body of the patient 10. A second fluid connection point is also provided, while not connected to an internal pipe in the scheme. A second regulating fluid can thus circulate in the casing or within the structural element to provide a different thermal effect to the patient **10.**

According to an embodiment, the casing connection panel **30** can be manufactured together with the structural element **21.** Alternatively, it is manufactured separately and integrated or combined to the structural element **21.**

According to another embodiment, the casing connection panel is adapted to connect individual connections means such as electrical, electronical, fluidic connection means and data exchange.

According to another embodiment, the casing connection panel 30 comprises at least one combined connection means adapted to plug several types of connection selected from an electrical, an electronical, a fluidic connection and data exchange.

In case more than on regulating fluid is used, they can be identical or different. One of the fluids can be well adapted for heat transfer while another one is well adapted for cooling effect. It is understood that the number and/or the type of connections is not limited and that several additional connection means can be envisaged depending on the needs.

Although the internal pipes **211** and electrical cables **212** are represented as being free, they can be embedded within the material of the structural element instead of crossing the internal hollow space **21d.** This applicable when the structural element is a casing **21C** or a skeleton **21S.**

The patient **10** can be the user of the system **1** here described. He can easily instal the element of contact **20** on a specific point of his body, connect at least one fluid connection point **220a, 220b** with dedicated pipes connected to the thermal regulation device **50** and activate the thermal regulation device **50** to obtain the expected thermal effect. The patient **10** can select a pre-recorded program so that the thermal regulation device **50** provides an adapted thermal effect. According to an embodiment, once a suitable temperature is obtained, the patient can disconnect the fluid connection point from the corresponding pipes so that he is free to move remote from the thermal regulation device **50** while the thermal effect operates through the element of contact **20.** According to another embodiment, the patient **10** can keep connected the fluid connection point so that an active thermal regulation is operated. The patient can select a program allowing to increase, decrease or maintain the temperature of the system **1,** and more particularly of the element of contact **20.** The patient can alternatively select a cyclic variation of the temperature. Alternatively, a patient can input some parameters to the thermal regulation device **50** according to specific needs. He can for example select a temperature to be maintained and controlled. He can select a lower and a high threshold of temperature, he can select an increase or a decrease rate of the temperature. He can select cyclic variations of temperatures including number of cycles and low and high temperature limits. Alternatively, the temperature can automatically vary or being adapted according to one or several values collected from one or more sensors. Such sensors include sensor for determining the temperature of the skin at the specific point of interest, the heart frequency, the blood pressure and any other suitable sensor or biosensor. According to a specific embodiment, several fluid regulations are circulating through the element of contact so has to provide different temperature at different specific points of the body. To this end, a given element of contact can be provided with several fluid connections so that several fluids can be connected. Alternatively, several elements of contact 20 are placed on the body at specific points of interest and each one of the elements of contact are connection to one thermal regulation device **50.** The temperature can thus be independently adapted to the area. The patient **10** can in addition select one or more effect from an electrical stimulation, a substance release, an infrared stimulation, an ultrasound stimulation, a magnetic stimulation, according to pre-recorded programs.

Other users than the patient **10** may be involved in the operation of the present system 1. For example, a remote user such as a medical agent, a sportive coach, a data analyst, a researcher or any other authorized person can receive data from the system 1. Such data may relate to the parameters collected by means of dedicated sensors such as heart frequency sensors, body temperature sensor and/or blood pressure sensors. The data can be sent through a data computing unit **40** or any suitable device adapted to collect and/or process at least partly the data.

According to an embodiment, the structural element **21** is made, at least partially, preferably in its entirety, of a rigid material, so that its shape is predefined and cannot be changed anymore. The shape of the surface of contact **21a** is thus adapted to fit with the portion of the body on which it is applied. In that case, the structural element can be made on demand, according to a fast and cheap process. For example, a rigid polymer can be processed through an additive manufacturing procedure so that it corresponds to the morphology of the patient. The additive manufacturing can be performed based on a numerical scan of the body to be considered, or any equivalent numerical recording means.

In practice, only the surface of contact **21a** may be processed through an additive manufacturing method. For example, in case the structural element is a casing as described above, part of the casing **21C,** comprising the edge **21c** and the top surface **21b** can be provided as a standard element, and based on this, the surface of contact **21a** having the dedicated shape can be added. The process is thus fast and cheap, in a way that it limits the processing efforts. Before adding the surface of contact **21a** to the rest of the casing **21C,** a pouch and/or other suitable elements, as needed, can be arranged in the casing if necessary.

Alternatively, the whole casing **21C** is made through an additive manufacturing operation on demand. This is more convenient in particular when very specific shapes need to be created.

In case the structural element is a skeleton **21S,** all or part of the skeleton can be made through an additive manufacturing process.

The process described here with the example of a casing **21C** having solid surfaces and a skeleton **21S,** it can be operated with any suitable structural element, whether it comprises beams or ribs, or grids.

According to a specific embodiment, not only the structural element is produced by means of an additive manufacturing process. For example, internal pipes can also be manufactured with the same process and during the same time so as to improve the time of manufacturing. According to an embodiment, the internal pipes are organized in a way to form, as such, a structural element. According to another embodiment, internal pipes are combined or integrated to a structural element. In case one or more reservoirs are considered, it can also be processed according to an additive manufacturing operation. A reservoir can be made in the same time or through a distinct process step compare to the production of the structural element. In case porous or semi-permeable element are needed, they can be produced according to a suitable additive manufacturing process, using either the same component or different components.

Although additive manufacturing is preferred compared to other known processes, this does not fully exclude that moulding and/or machining some parts or all of the structural element can still be considered.

According to another embodiment, the structural element **21** is semi-rigid, meaning that it can be modelled by hand while keeping the shape one has urged it to adopt. The structural element **21** can therefore by made, at least partly, in soft polymer adapted to be modelled by hand. In this case, the structural element **21** can be modelled by the patient **10** himself in a way to be adjusted to his morphology at the specific point of his body. In some extend, the surface of contact **21a** can be the only part that is semi-rigid while the remaining parts of the structural element **21** is rigid. Alternatively, the whole structural element **21** can be made of a semi-rigid material. The surface of contact **21a** or the whole structural element **21** can take the suitable shape by pressing it at the specific point of body so that the surface of contact **21a** takes the suitable complementary shape.

Rigid or semi-rigid structural element is applicable independently of whether it comprises only solid surfaces, and/or beams and/or ribs and/or grid.

The structural element **21** can be coated, at least on the surface of contact **21a,** with one or several soft materials such as a silicon layer or a rubber layer or any suitable soft material. This is particularly suitable in case the structural element is directly applied on the body.

In a preferred arrangement, the structural element, or a combination of several structural elements, is included or inserted into an external pouch **80.** Such an external pouch **80** is preferably used to improve the comfort of use of the element of contact **20.** The external pouch **80** can be made for example of a fabric, of a textile made of natural fibres or synthetic fibres or both, of a soft polymer or mixture of polymers, on a natural polymer such as rubber, or of any other suitable material.

In addition, the external pouch can comprise additional materials, used as a filler **81** such as an internal gel or internal bids of material surrounding the structural element 21. This additional internal material provides the necessary flexibility and contact sensation to the element of contact, which is thus well adapted. The additional internal material can be selected in addition to improve the storage or the release of thermal energy.

It is understood that all the features and arrangement described here can be used independently or combined where applicable.

The present disclosure provides also a process for the manufacture of a structural element **21,** or an assembly of combined structural elements. The process comprises a step of determining or selecting at least one specific point of body. The patient **10** may himself determine the location of the pain his suffers from, or the location which needs a thermal effect. He may also determine himself the nature of his pain. The nature can for example relate to an inflammation or aches following unusual physical efforts he is aware of, or to a sprain following a wrong move he has made. Alternatively, a professional practitioner such as a doctor or a medical specialist can diagnose the origin, the precise location and/or the nature of his pain.

The process further comprises a step of determining the tridimensionality shape of the body at the specific point of interest. Although a mould such as plaster moulded may be considered, the tridimensionality shape of the specific point of body is preferably obtained through a scanning operation, so as to obtain precise numerical data related to the shape of the body at this location.

Once the tridimensionality shape is known, the process comprises the step of determining a suitable tridimensionality shape for an element of contact **20.** Determining the suitable shape includes not only determining the tridimensionality shape of the surface of contact **21a,** but also the necessity to design one or more subparts **20a, 20b.** The tridimensionality shape of the structural element can be deduced from the step of determining the tridimensionality shape of the element of contact. Alternatively, the tridimensionality shape of the structural element **21** can be directly determined from the tridimensionality shape of the specific point of body. The process may also include a step of determining the nature of the structural element or the structural elements **21.** The nature includes for example the selection of solid surface, or beams, or grids, or a combination thereof. It can thus be decided to use a casing **21C** or a skeleton **21S** or an alternative structural element. The nature also includes whether internal pipes are integrated, or fused or combined with the structural element, or on contrary separately designed. The nature includes the presence or not of one or several reservoirs. The nature also includes the type of material used for the structural element and whether it should be semi-rigid or rigid. Once the suitable tridimensionality shape and the nature of the structural element or the structural elements **21** is determined, the process includes a step of manufacturing the structural element or the structural elements **21,** including those related to the different subparts **20a, 20b.** The manufacturing preferably includes or consists of an additive manufacturing. An additive manufacturing also denotes 3D printing operations and related processes.

According to a specific embodiment, the thermal regulation device 50 can be provided with means of pressurisation. In particular, the pouch may be inflated with pressurized air.

### Reference symbols in the figures

- 10: Patient
- 20: Element of contact
- 20a, 20b: Subparts of element of contact
- 21: Structural element
- 21C: Casing
- 21S: Skeleton
- 21a: Surface of contact
- 21b: Top surface
- 21c: Edge
- 21d: Internal hollow space
- 210: Electrical connection means of element of contact
- 231a, 231b: Fluid pins for cream, liquid, gel connections
- 210c, 210d: Electrical pins for electro-stimuli connections
- 211: Internal pipes for cream, liquid, gel delivery
- 212: Electrical cables
- 220a,220b: First fluid connection points
- 221: Internal pipes for hot or cold fluid
- 230: Reservoir
- 240a, 240b: Porous areas
- 241a, 241b, 241c, 241d: Stimulation plates
- 30: Casing connection panel
- 40: Data computing unit
- 50: Regulation device
- 51: Support
- 52a, 52b: Pairs of wheels
- 53: Solar panel
- 54: Air-exchange system
- 55: Display
- 56: Connection panel
- 57: Command panel
- 58a, 58b: Thermal regulation elements
- 59: Electrical stimulation system
- 562: 230V Electrical plug
- 563: 24V plug
- 564a, 564b, 565a, 565b: Second fluid connection point
- 566, 567: Electrical connection means of regulation device
- 568: USB
- 561: 12V plug
- 60: Circulating fluid
- 70: Pump
- 80: Pouch
- 81: Filler

## Claims

1. System (1) adapted for providing a thermal and/or therapeutical effect at a specific point of body of a patient (10) comprising :
- one or more element of contact (20a, 20b), adapted to be placed in contact or close to said specific point of body, said one or more element of contact comprising at least one first fluid connection point (220a, 220b) adapted to circulate a regulation fluid (60) through said one or more element of contact,
- a thermal regulation device (50), comprising at least one pump (70) and at least one second fluid connection point (564a, 564b, 565a, 565b),
- at least one regulation fluid (60) whose temperature can be regulated by said thermal regulation device (50),
- at least one dissociated or integrated casing connection panel (30) related to structural element (21)
- one or several pipes, fluidly connecting said first fluid connection point (220a, 220b) to said second fluid connection point (564a, 564b, 565a, 565b),
wherein the at least one regulation fluid (60) can be circulated through the element of contact (20) by means of the at least one pump (70).

2. System according to claim 1, wherein said element of contact (20) comprises a rigid or semi-rigid casing (21C, 21S) as structural element (21), said element having a tridimensionality shape which fits the morphology of said patient at the specific point of body.

3. System according to claim 1, wherein said element of contact (20) comprises a flexible pouch (80) and a rigid or semi rigid skeleton (21S) as structural element for giving to said pouch a tridimensionality shape which fits the morphology of said patient at the specific point of body.

4. System according to one of the claims 2 to 3, further comprising a filler (81) filling said casing respectively said pouch.

5. System according to one of claims 2 to 4, further comprising internal pipes (221) adapted to circulate at least one fluid through the element of contact (20).

6. System according to claim 5, said internal pipes being combined or integrated to, or forming at least part of the structural element.

7. System according to one of claims 1 to 6, wherein said element of contact (21) further comprises at least one additional stimulation device selected from an electrical stimulation device, a magnetic stimulation device, an infrared emitting device.

8. System according to one of claims 1 to 7, wherein the system further comprises or is connectable to at least one sensor selected among a thermo-sensor, heart frequency sensor, blood pressure sensor or at least one sensor related to the usage of the compress.

9. System according to one of claims 1 to 8, wherein said element of contact (20) further comprises one or several reservoirs (230) for a drug or essential oils to be administrated to said patient.

10. System according to one of claims 1 to 9, wherein said at least one regulation fluid is a liquid or a gel.

11. System according to one of claims 1 to 10, wherein the thermal regulation device (50) comprises one or both of a heat production element (58a) in a range of temperature between 35°C to 45°C and/or a cooling element (58b) in a range of temperature between - 5° to 5°.

12. System according to claim 11, comprising a cool fluid circulating circuit and a warm fluid circulating circuit.

13. System according to one of claims 1 to 12, wherein said thermal regulation device (50) is adapted for creating cycles of increasing and decreasing temperature of said regulation fluid during application to one or several patients.

14. System according to one of the claims 1 to 13, said thermal regulation device being adapted to apply a pre-recorded program among a menu and/or to determine parameters selected from temperature, temperature ramp, thresholds values of temperatures, flowrate of the regulating liquid, additional electrical stimulation, additional ultrasound stimulation, additional magnetic stimulation, infrared irradiation.

15. System according to one of the claims 1 to 14, said structural element being a 3D printed element.

16. Process for the manufacture of structural element of an element of contact (20) as described in claims 2 to 11, said structural element being rigid or a semi-rigid (21) and comprising at least a surface of contact (21a) having a tridimensionality shape complementary of the tridimensionality shape of a specific point of a patient's body, said process comprising the steps of determining the morphology of said patient, the step of determining the tridimensionality shape of a structural element (21) according to the morphology of said patient, and a step of 3D printing the structural element (21), so that the tridimensionality shape of the surface of contact (21a) fits with the morphology of the patient.
